# FASCICULE DE BREVET EUROPEEN

(11) **EP 1 136 058 B1**
(45) Date de publication et mention de la délivrance du brevet: **03.05.2006**
(21) Numéro de dépôt: 01400373.5
(22) Date de dépôt: 13.02.2001
(51) Int. Cl.: A61K 8/06, A61K 8/894, A61K 8/81, C08J 3/09, C08L 83/12

(54) **Composition sous forme d'emulsion eau-dans-huile et ses utilisations cosmétiques**
Zusammensetzung vom Typ-Wasser-in-Öl Emulsion und ihre Verwendung in der Kosmetik
Water-in-oil type emulsioned composition and cosmetic uses thereof

(30) Priorité: 21.03.2000 FR 0003589
(43) Date de publication de la demande: 26.09.2001
(73) Titulaire: L'OREAL, 75008 Paris (FR)
(72) Inventeur: Lorant, Racula, 94320 Thiais (FR)
(74) Mandataire: Rasson, Catherine

(56) Documents cités:
- EP-A- 1 055 406
- US-A- 5 262 087
- US-A- 5 412 004
- US-A- 5 470 551
- PATENT ABSTRACTS OF JAPAN vol. 1999, no. 09, 30 juillet 1999 (1999-07-30) & JP 11 092335 A (KOSE CORP), 6 avril 1999 (1999-04-06)
- PATENT ABSTRACTS OF JAPAN vol. 1999, no. 04, 30 avril 1999 (1999-04-30) & JP 11 021227 A (KOSE CORP), 26 janvier 1999 (1999-01-26)

## Description

L'invention se rapporte à une composition se présentant sous forme d'une émulsion eau-dans-huile (E/H) comportant un organopolysiloxane élastomère réticulé oxyalkyléné et un polymère particulier, et à ses utilisations notamment dans les domaines cosmétique et/ou dermatologique, en particulier comme produits de soin, de nettoyage et/ou de maquillage de la peau.

Dans les domaines cosmétique ou dermatologique, il est courant d'utiliser des compositions ayant l'aspect d'une crème et constituées d'une émulsion eau-dans-huile (E/H) comportant une phase aqueuse dispersée dans une phase huileuse. Une crème est, dans les domaines considérés, une composition présentant une certaine viscosité, par opposition aux compositions liquides ou semi-liquides telles que les lotions et les laits, ou encore aux compositions solides.

Les émulsions E/H comportent une phase continue huileuse et permettent donc de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches.

Toutefois, les crèmes sous forme d'émulsions E/H présentent l'inconvénient d'être inconfortables du fait de la sensation grasse, lourde et parfois même collante, apportée par cette phase grasse externe qui subsiste sur la peau. Ainsi, ces crèmes sont en général utilisées pour les peaux sèches, étant trop grasses pour être utilisées sur les peaux grasses. De plus, elles n'apportent pas de fraîcheur et sont généralement trop riches en huiles pour être utilisées pendant l'été ou dans les pays chauds.

Pour surmonter ces inconvénients, il a été envisagé de préparer des émulsions E/H à forte teneur en eau. Toutefois, la teneur en eau ne peut pas être trop importante pour des raisons de stabilité, ou alors une forte teneur en eau doit être compensée par l'ajout de plusieurs tensioactifs qui peuvent nuire au confort de la composition finale et même entraîner des problèmes d'irritations cutanés notamment chez les sujets à peaux sensibles, et/ou par l'ajout d'électrolytes qui ont l'inconvénient d'être incompatibles avec un nombre important d'ingrédients et principes actifs que l'on utilise dans les compositions cosmétiques et dermatologiques.

Il subsiste donc le besoin d'une composition sous forme d'une émulsion eau-dans-huile utilisable dans les domaines cosmétique et/ou dermatologique, qui ne présente pas les inconvénients de l'art antérieur et notamment qui procure de la fraîcheur sans apport d'effet gras ou collant lors de l'application sur la peau, tout en ayant une bonne stabilité. On entend ici par « émulsion de bonne stabilité » une émulsion qui soit homogène et régulière, et qui ne se déphase (séparation de la phase aqueuse et de la phase huileuse) ni ne relargue d'huile, au moins pendant deux mois à 45°C.

La demanderesse a maintenant trouvé de manière surprenante que l'on pouvait obtenir une composition du type émulsion eau dans huile permettant d'atteindre étant très stable, en utilisant l'association d'un organopolysiloxane élastomère réticulé oxyalkyléné et d'un polymère poly(acide 2-acrylamido 2-méthylpropane sulfonique) réticulé et au moins partiellement neutralisé.

L'invention a pour objet une composition comprenant, dans un milieu physiologiquement acceptable, une phase aqueuse dispersée dans une phase huileuse, caractérisée en ce que la phase huileuse comprend des particules d'un organopolysiloxane solide élastomère réticulé comportant au moins un groupement oxyalkyléné et en ce que la phase aqueuse comprend au moins un polymère poly(acide 2-acrylamido 2-méthylpropane sulfonique) réticulé et neutralisé.

Le document EP-A-1,055,406 qui fait partie de l'état de la technique au sens de l'article 54(3) CBE décrit des émulsions' huile-dans-eau comportant un organopolysiloxane solide élastomère réticulé comportant au moins un groupement oxyalkyléné et (ii) un polymère hydrosoluble ou gonflable dans l'eau.

On entend par milieu physiologiquement acceptable dans la présente invention, un milieu non toxique, compatible avec la peau (y compris l'intérieur des paupières), les muqueuses, les cheveux ou les lèvres d'êtres humains.

La composition de l'invention a l'avantage d'être homogène et de rester stable dans le temps, même quand elle contient une quantité importante de phase aqueuse interne. En outre, elle possède l'avantage de ne pas nécessiter l'ajout d'électrolytes habituellement utilisés pour stabiliser les émulsions E/H. Elle peut donc être avantageusement exempte d'électrolytes.

Ainsi, l'invention a aussi pour objet l'utilisation de l'association de particules d'un organopolysiloxane solide élastomère réticulé comportant au moins un groupement oxyalkyléné et d'au moins un polymère poly(acide 2-acrylamido 2-méthylpropane sulfonique) réticulé et neutralisé, pour obtenir une émulsion eau-dans-huile stable.

L'invention a aussi pour objet l'utilisation de l'association de particules d'un organopolysiloxane solide élastomère réticulé comportant au moins un groupement oxyalkyléné et d'au moins un polymère poly(acide 2-acrylamido 2-méthylpropane sulfonique) réticulé et neutralisé, comme agent stabilisant d'une émulsion eau-dans-huile.

Par ailleurs, la composition de l'invention a l'avantage d'être très fraîche et non collante lors de l'application sur la peau, tout en ayant un toucher très confortable (douceur, absence d'irritation).

L'organopolysiloxane solide élastomère réticulé comportant au moins un groupement oxyalkyléné utilisé dans la composition de l'invention permet d'assurer la dispersion de la phase aqueuse dans la phase huileuse de l'émulsion.

Par « solide élastomère », on entend ici un matériau souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes de la composition de l'invention contiennent un ou plusieurs groupements oxyalkylénés et en particulier oxyéthylénés (OE), par exemple de 1 à 40 motifs oxyalkylénés, de préférence de 1 à 20, mieux de 10 à 20, de façon plus préférée de 12 à 20 et encore mieux de 12 à 18 motifs oxyalkylénés, pouvant former des chaînes polyoxyalkylènes et notamment polyoxyéthylènes. Ces groupements peuvent être pendants, en bout de chaîne ou destinés à lier deux parties de la structure siliconée. Les atomes de silicium portant ces groupements sont avantageusement au nombre d'environ 1 à 10 et mieux de 1 à 6.

Bien que l'invention concerne plus spécialement les organopolysiloxanes à groupement(s) oxyéthyléné(s) (à savoir les groupements ne comportant que des groupements oxyéthylénés comme groupements oxyalkylénés), elle peut aussi concerner les organopolysiloxanes à groupement(s) oxypropyléné(s), c'est-à-dire ne comportant que des groupements oxypropylénés comme groupements oxyalkylénés. Les organopolysiloxanes peuvent aussi comporter à la fois un ou plusieurs groupement(s) oxyéthyléné(s), 1 à 20 (OE) par exemple, et un ou plusieurs groupement(s) oxypropyléné(s) (OP), 0 à 20 par exemple ; ces organopolysiloxanes sont appelés aussi des organopolysiloxanes à groupement(s) alkyléthoxy-propyléné(s). De préférence, le nombre de groupements oxyéthylénés est supérieur au nombre de groupements oxypropylénés.

Par ailleurs, la structure siliconée formant le squelette polymérique de l'organopolysiloxane à groupement(s) oxyalkyléné(s) est avantageusement une structure polydiméthylsiloxane (PDMS) dont éventuellement une partie des groupes méthyle est substituée par des groupements alkyle en C₂ à C₃₀ et de préférence en C₈ à C₂₄, et mieux de C₁₀ à C₂₀ ou phényle, soit en bout de chaîne soit pendants.

Par ailleurs, l'organopolysiloxane à groupement(s) oxyalkyléné(s) peut comporter un ou plusieurs squelette(s) siliconé(s) relié(s) entre eux par un ou plusieurs groupements oxyalkylénés et de préférence oxyéthylénés tels que définis précédemment, ou par un ou plusieurs groupements alkylénés, le nombre de groupement alkyléné allant de 1 à 30 et de préférence de 1 à 20. De préférence, il comporte au moins deux squelettes polymériques liés entre eux. Avantageusement, le ou les squelettes siliconés des organopolysiloxanes de la composition selon l'invention comportent de 26 à 80 atomes de silicium.

Les organopolysiloxanes élastomères utilisés dans la composition conforme à l'invention sont partiellement ou totalement réticulés et de structure tridimensionnelle. Inclus dans une phase huileuse, ils se transforment, selon le taux de phase huileuse utilisé, d'un produit d'aspect spongieux lorsqu'ils sont utilisés en présence de faibles teneurs en phase huileuse, en un gel homogène en présence de quantités de phase huileuse plus élevées. La gélification de la phase huileuse par ces élastomères peut être totale ou partielle.

Ces organopolysiloxanes élastomères peuvent se présenter sous forme de poudre, les particules constituant cette poudre ayant une taille allant généralement de 0,1 à 500 µm, de préférence de 3 à 200 µm et mieux de 3 à 50 µm, et pouvant être sphériques, plates ou amorphes avec, de préférence, une forme sphérique. Ils peuvent aussi se présenter sous forme de gel anhydre contenant l'organopolysiloxane élastomère dispersé dans une phase huileuse. Cette phase huileuse, appelée encore phase grasse liquide, peut comprendre tout corps non aqueux ou mélange de corps non aqueux, liquide à température ambiante (environ 25°C) et à la pression atmosphérique (760 mm de Hg).

Les organopolysiloxanes élastomères utilisés selon l'invention peuvent être choisis parmi les polymères réticulés obtenus par réaction d'addition et de réticulation en milieu non aqueux, en présence d'un catalyseur notamment du type platine, d'au moins :
- (a) un premier organopolysiloxane (i) ayant au moins deux groupes vinyliques en position α-ω de la chaîne siliconée ; et
- (b) un second organopolysiloxane (ii) ayant au moins un atome d'hydrogène lié à un atome de silicium par molécule et au moins un groupement oxyalkyléné notamment oxyéthyléné.

En particulier, l'organopolysiloxane (i) est choisi parmi les polydiméthylsiloxanes (PDMS) et est plus spécifiquement un α-ω-diméthylvinyl polydiméthylsiloxane. L'organopolysiloxane (ii) est choisi notamment parmi les polydiméthylsiloxanes comportant un ou plusieurs atome(s) d'hydrogène, lié chacun à un atome de silicium, et un ou plusieurs groupements oxyéthylénés et éventuellement un ou plusieurs groupements oxypropylénés, liés à un atome de silicium via un radical alkylène ayant de 1 à 22 atomes de carbone.

Eventuellement les chaînes silicones des premiers et seconds organopolysiloxanes (i) et (ii) comportent des chaînes pendantes alkyle en C₁ à C₆ et/ou des chaînes aryle.

Comme indiqué ci-dessus, les organopolysiloxanes élastomères utilisables dans la composition selon l'invention se présentent avantageusement dans une phase huileuse avec laquelle ils constituent un gel anhydre. Ce gel peut être notamment obtenu comme suit :
- (a) mélange du premier organopolysiloxane (i) et du second organopolysiloxane (ii) ;
- (b) ajout d'une phase huileuse au mélange de l'étape (a) ; et
- (c) polymérisation du premier organopolysiloxane (i) et du second organopolysiloxane (ii) en phase huileuse en présence d'un catalyseur de platine.

La phase huileuse utilisée lors de la fabrication du gel anhydre contient une ou plusieurs huiles liquides à températures ambiante (environ 25°C) choisies parmi les huiles hydrocarbonées et/ou les huiles de silicone. Avantageusement, la phase huileuse est une phase liquide siliconée, contenant une ou plusieurs huiles choisies parmi ies polydiméthylsiloxanes (PDMS) à chaîne linéaire ou cyclique, liquides à température ambiante et comportant éventuellement une chaîne alkyle ou aryle pendante ou en bout de chaîne, la chaîne alkyle ayant de 1 à 6 atomes de carbone.

Les organopolysiloxanes de l'invention sont en particulier obtenus selon le mode opératoire des exemples 3, 4 et 8 du document US-A-5,412,004 et des exemples du document US-A-5,811,487.

Les organopolysiloxanes de la composition de l'invention sont par exemple celui commercialisé sous la référence KSG 21 par la société Shin Etsu ou le produit de l'exemple 3 (exemple de synthèse) du brevet US-A-5,412,004.

Le KSG 21 se présente sous forme d'un gel pâteux comprenant environ 28 % d'organopolysiloxane réticulé à groupement(s) oxyéthyléné(s) et 72 % d'huile de silicone (PDMS) ayant une viscosité de 6 cSt (soit 6.10⁻⁶ m²/s).

Le produit de l'exemple 3 (exemple de synthèse) du document US-A-5,412,004 se présente sous la forme d'un gel pâteux contenant environ 33 % en poids d'organopolysiloxane réticulé à groupement(s) oxyéthyléné(s) et environ 67 % de PDMS 6 cSt (soit 6.10⁻⁶ m²/s). L'organopolysiloxane contient environ 18 % d'oxyde d'éthylène en poids par rapport au poids total du polymère.

Le gel élastomère de l'invention a un comportement rhéologique plastique présentant une viscosité sous faible cisaillement voisin de 10⁻³ s⁻¹ ou 10⁻⁴ s⁻¹, allant de 2.10⁶ Poises à 4.10⁶ Poises (2.10⁵ Pa.s à 4.10⁵ Pa.s), et une viscosité dynamique de 15 à 50 Poises (1,5 à 5 Pa.s) pour une vitesse de cisaillement de 200 s⁻¹ à t_{10 minutes}, mesurée avec un rhéomètre à contrainte imposée, RS 75 (Haake) à 25°C en géométrie cône/plan ; caractéristique du cône : 20 mm de diamètre, 1° d'angle et 40 µm de gap. Cet organopolysiloxane a, en outre, un comportement viscoélastique, avec un caractère élastique dominant aux faibles valeurs de la contrainte de cisaillement, défini comme suit : 800 Pa < G * ₚₗₐₜₑₐᵤ < 2.500 Pa avec δ ₚₗₐₜₑₐᵤ voisin de 10°, G* ₚₗₐₜₑₐᵤ représentant la consistance et δ ₚₗₐₜₑₐᵤ représentant l'élasticité, cette mesure étant faite à 1 Hz. Il présente un point éclair d'environ 170°C à la pression atmosphérique.

Pour le produit de l'exemple 3 (exemple de synthèse) du document US-A-5,412,004, la viscosité dynamique, dans les conditions indiquées ci-dessus, est de 45 Poises (4,5 Pa.s).

De façon préférentielle, le gel d'organopolysiloxane élastomère est présent dans la composition de l'invention en une quantité allant de 0,3 à 30 % en poids, et mieux de 1,5 à 18 % en poids par rapport au poids total de la composition, ce qui correspond à un taux d'organopolysiloxane élastomère en matière active allant de préférence de 0,1 à 10 % en poids et mieux de 0,5 à 6 % en poids par rapport au poids total de la composition.

L'organopolysiloxane élastomère de l'invention est en particulier un tensioactif de HLB (Balance Hydrophile Lipophile) d'environ 2,5.

La phase huileuse de la composition selon l'invention peut renfermer, outre l'huile éventuellement présente dans le gel anhydre en mélange avec l'organopolysiloxane élastomère réticulé, toutes les sortes d'huiles et de corps gras bien connus de l'homme du métier, comme par exemple les huiles d'origine végétale (jojoba, avocat, sésame, tournesol, maïs, soja, carthame, pépins de raisin), les huiles minérales (vaseline, isoparaffines), les huiles de synthèse (myristate d'isopropyle, octanoate de cétéaryle, polyisobutylène, palmitate d'éthyl-hexyle, alkyl benzoates), les huiles de silicone volatiles ou non volatiles telles que les polydiméthylsiloxanes et les silicones cycliques (cyclodiméthylsiloxanes ou cyclométhicones), et les huiles fluorées ou fluorosiliconées, ainsi que les mélanges de ces huiles.

De préférence, la phase huileuse de la composition de l'invention comprend au moins une huile volatile en une quantité d'au moins 1 % en poids et allant de préférence de 2 à 35 % en poids et mieux de 5 à 25 % en poids par rapport au poids total de la composition. Par huile volatile, on entend en particulier une huile susceptible de s'évaporer, en moins d'une heure, au contact de la peau ou des lèvres, ayant notamment une pression vapeur non nulle, en particulier allant de 10⁻³ à 300 mm de Hg (à température ambiante et pression atmosphérique) et de préférence supérieure à 0,3 mm de Hg. L'huile volatile peut être notamment choisie parmi les huiles hydrocarbonées (c'est-à-dire ne comportant que des carbones et des hydrogènes) à chaîne ramifiée telles que l'isohexadecane et l'isododecane, et parmi les huiles de silicone volatiles (c'est-à-dire les huiles de silicone ayant une viscosité inférieure à 8 cSt), telles que par exemple les silicones cycliques (cyclométhicones) comme la cyclopentaméthicone, la cyclotétraméthicone, la cyclohexaméthicone, et leurs mélanges.

La phase huileuse peut contenir, en outre d'autres constituants gras tels que les alcools gras comme l'alcool stéarylique, l'alcool cétylique et l'alcool cétéarylique, et les acides gras.

Quand la composition est utilisée comme composition démaquillante, notamment pour le démaquillage de la peau et/ou des yeux, elle peut comprendre de manière avantageuse une huile démaquillante. L'huile démaquillante peut être choisie notamment parmi les esters d'acide gras comportant au moins 12 atomes de carbone et de préférence ceux obtenus à partir d'un alcool à chaîne droite ou ramifiée, comportant de 1 à 17 atomes de carbone et d'un acide gras à chaîne droite ou ramifiée, comportant au moins 12 atomes de carbone et de préférence de 14 à 22 atomes de carbone. Il s'agit de préférence de mono- ou de di-esters. De manière préférée, ces esters ne comportent aucune insaturation et/ou aucun groupement éther ou hydroxyle. De façon encore plus avantageuse, il s'agit d'un ester saturé qui ne renferme aucun groupement éther ni hydroxyle.

Comme ester d'acide gras utilisable comme huile démaquillante, on peut citer notamment le palmitate d'éthyl-2 hexyle (ou palmitate d'octyle), le myristate d'éthyl-2 hexyle (ou myristate d'octyle), le palmitate d'isopropyle, le myristate d'isopropyle, l'adipate de di-isopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, et leurs mélanges.

La phase huileuse est présente dans la composition selon l'invention en une quantité allant de 5 à 50 % et de préférence de 5 à 25 % en poids par rapport au poids total de la composition.

Les polymères poly(acide 2-acrylamido 2-méthylpropane sulfoniques) utilisables dans la composition de l'invention sont réticulés et neutralisés. On entend dans la présente invention par « neutralisés » des polymères totalement ou pratiquement totalement neutralisés, c'est-à-dire au moins neutralisés à 90 %. Ces polymères sont hydrosolubles ou gonflables dans l'eau. Ils sont en général caractérisés par le fait qu'ils comprennent, distribués de façon aléatoire :
a) de 90 à 99,9% en poids de motifs de formule générale (I) suivante : 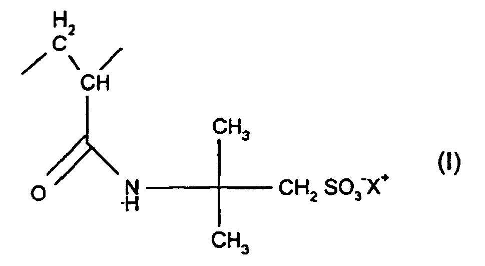 dans laquelle X⁺ désigne un cation ou un mélange de cations, au plus 10% mol des cations X⁺ pouvant être des protons H⁺ ;
b) de 0,01 à 10% en poids de motifs réticulants provenant d'au moins un monomère de réticulation ayant au moins deux doubles liaisons oléfiniques ; les proportions en poids étant définis par rapport au poids total du polymère.

De façon préférentielle, les polymères de invention comportent un nombre de motifs de formule (I) dans une quantité suffisamment élevée pour obtenir des particules de polymère dont le volume hydrodynamique en solution d'eau présente un rayon allant de 10 à 500 nm et dont la distribution est homogène et unimodale.

Les polymères selon l'invention plus particulièrement préférés comprennent de 98 à 99,5 % en poids de motifs de formule (I) et de 0,2 à 2 % en poids de motifs réticulants.

Dans la formule (I), X⁺ représente un cation ou un mélange de cations choisis en particulier parmi un proton, un cation de métal alcalin, un cation équivalent de celui d'un métal alcalino-terreux ou l'ion ammonium.

Plus particulièrement, 90 à 100% mole des cations sont des cations NH₄⁺ et 0 à 10% mole sont des protons (H⁺).

Les monomères de réticulation ayant au moins deux doubles liaisons oléfiniques sont choisis par exemple parmi le dipropylèneglycol-diallyléther, les polyglycol-diallyléthers, le triéthylèneglycol-divinyléther, l'hydroquinone-diallyl-éther, le tétrallyl-oxéthanoyle ou d'autres allyl ou vinyléthers d'alcools polyfonctionnels, le diacrylate de tétraéthylèneglycol, la triallylamine, le triméthylolpropane-diallyléther, le méthylene-bis-acrylamide ou le divinylbenzène.

Les monomères de réticulation ayant au moins deux doubles liaisons oléfiniques sont plus particulièrement choisis parmi ceux répondant à la formule générale (II) suivante : 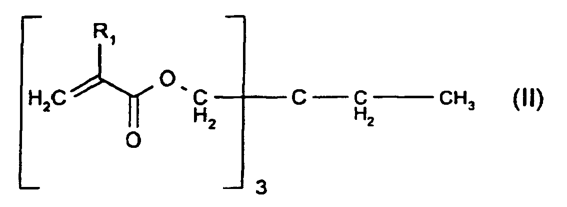 dans laquelle R₁ désigne un atome d'hydrogène ou un alkyle en C₁-C₄ et plus particulièrement méthyle. Le monomère de réticulation est de préférence le triméthylol propane triacrylate (composé de formule II où R₁ est l'hydrogène).

Les polymères particulièrement préférés sont ceux présentant une viscosité mesurée au viscosimètre BROOKFIELD, mobile 4, à une vitesse de rotation de 100 tours/minute dans une solution d'eau à 2 % et à une température d'environ 25°C, supérieure ou égale à 1000 cPs (ou 1000 mPa.s) et plus préférentiellement allant de 5000 à 40.000 cPs (5000 à 40.000 mPa.s) et plus particulièrement de 6500 à 35.000 cPs (6500 à 35.000 mPa.s).

Les poly(acide 2-acrylamido 2-méthylpropane sulfonique) réticulés et neutralisés utilisés dans la composition de l'invention peuvent être obtenus selon le procédé de préparation comprenant les étapes suivantes :
(a) on disperse ou on dissout le monomère acide 2-acrylamido 2-méthylpropane sulfonique sous forme libre dans une solution de tertio-butanol ou d'eau et de tertio-butanol;
(b) on neutralise la solution ou la dispersion de monomère obtenue en (a) par une ou plusieurs bases minérales ou organiques, de préférence l'ammoniaque NH₃, dans une quantité permettant d'obtenir un taux de neutralisation des fonctions acides sulfoniques du polymère allant de 90 à 100% ;
(c) on ajoute à la solution ou dispersion obtenue en (b), le ou les monomères réticulants ;
(d) on effectue une polymérisation radicalaire classique en la présence d'amorceurs de radicaux libres à une température allant de 10 à 150°C ; le polymère précipitant dans la solution ou la dispersion à base de tertio-butanol.

Le poly(acide 2-acrylamido 2-méthylpropane sulfonique) utilisé dans la composition de l'invention peut être notamment le produit commercialisé par la société Hoechst sous la dénomination commerciale « Hostacerin AMPS » (nom CTFA : ammonium polyacryldimethyltauramide).

Le poly(acide 2-acrylamido 2-méthylpropane sulfonique) réticulé et neutralisé, utilisé dans la composition de l'invention est de préférence présent en une quantité allant de 0,1 à 10 % en poids de matière active, mieux de 0,2 à 5 % en poids et plus préférentiellement de 0,5 à 3 % en poids par rapport au poids total de la composition.

Selon un mode particulier de réalisation de l'invention, la composition selon l'invention comporte au moins 65 % en poids de phase aqueuse par rapport au poids total de la composition et de préférence au moins 75 % du poids total de la composition en vue d'apporter un maximum de fraîcheur. La phase aqueuse peut constituer jusqu'à 90 % du poids total de la composition.

Selon un mode préféré de réalisation de l'invention, l'eau constitue au moins 40 % et de préférence au moins 50 % du poids total de la composition.

La composition selon l'invention constitue de préférence une composition cosmétique. On entend par « composition cosmétique » un produit ayant un aspect, une odeur et un toucher agréables, et destiné à une application topique. Cette composition trouve son application dans un grand nombre de traitements notamment cosmétiques de la peau, y compris du cuir chevelu, des cheveux, des ongles, et/ou des muqueuses, en particulier pour le soin, le nettoyage et/ou le maquillage et/ou la protection solaire de la peau et/ou des muqueuses, ainsi que pour la préparation d'une crème destinée au traitement de la peau, plus particulièrement de la peau grasse (apport de fraîcheur).

Aussi, la présente invention a pour objet l'utilisation cosmétique de la composition telle que définie ci-dessus, pour le traitement, la protection, le soin, le démaquillage et/ou le nettoyage de la peau, des lèvres et/ou des cheveux, et/ou pour le maquillage de la peau et/ou des lèvres.

La présente invention a encore pour objet un procédé de traitement cosmétique de la peau, y compris du cuir chevelu, des cheveux, et/ou des lèvres, caractérisé par le fait que l'on applique sur la peau, les cheveux et/ou les lèvres, une composition telle que définie ci-dessus.

L'invention a aussi pour objet l'utilisation de la composition telle que définie ci-dessus pour la fabrication d'une crème destinée au traitement des peaux grasses.

De façon connue, la composition de l'invention peut contenir également des adjuvants habituels dans les domaines cosmétique et/ou dermatologique, tels que les actifs, les conservateurs, les antioxydants, les agents complexants, les solvants, les parfums, les charges, les bactéricides, les absorbeurs d'odeur, les matières colorantes (colorants et pigments) et encore les vésicules lipidiques. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les vésicules lipidiques.

Comme solvants hydrophiles, on peut citer par exemple les alcools inférieurs comportant 2 à 8 atomes de carbone, comme l'éthanol et l'isopropanol, et les polyols comme la glycérine et les glycols.

Comme charges pouvant être utilisées dans la composition de l'invention, on peut citer par exemple, les poudres de matériaux organiques naturels tels que les amidons de maïs, de blé ou de riz, réticulés ou non, notamment les poudres d'amidon réticulé par l'anhydride octénylsuccinate, commercialisées sous la dénomination DRY-FLO par la société National Starch.

La composition est de préférence exempte d'actif instable en milieu oxydant en une teneur de 0,01 à 20 % du poids de la composition.

Aussi, l'invention a encore pour objet une composition comprenant, dans un milieu physiologiquement acceptable, une phase aqueuse dispersée dans une phase huileuse, caractérisée en ce que la phase huileuse comprend des particules d'un organopolysiloxane solide élastomère réticulé comportant au moins un groupement oxyalkyléné et en ce que la phase aqueuse comprend au moins un polymère poly(acide 2-acrylamido 2-méthylpropane sulfonique) réticulé et neutralisé, la dite composition étant exempte d'actif instable en milieu oxydant en une teneur de 0,01 à 20 % du poids total de la composition.

Les exemples ci-après de compositions selon l'invention sont donnés à titre d'illustration et sans caractère limitatif. Les quantités y sont données en % en poids, sauf mention contraire.

### Exemple 1 : crème pour le corps

### A. Phase huileuse

- Cyclohexaméthicone 15 %
- Silicone modifiée (exemple 3 du brevet US-5,412,004) 8 %
   (soit 2,64 % de matière active)

### B. Phase aqueuse

- Glycérine 5 %
- Hostacerin AMPS 1 %
- Eau 71 %

Mode opératoire : on prépare séparément les deux phases et on introduit la phase aqueuse gélifiée dans la phase huileuse sous agitation.

On obtient une crème blanche très stable dans le temps et conférant une grande sensation de fraîcheur lors de l'application sur la peau.

### Exemple 2 : crème pour le visage

### A. Phase huileuse

- Cyclohexaméthicone 10 %
- Isohexadecane 5 %
- Silicone modifiée (exemple 3 du brevet US-5,412,004) 3 %
   (soit 0,99 % de matière active)

### B. Phase aqueuse

- Hostacerin AMPS 1 %
- Conservateur 0,4 %
- Eau qsp 100 %

Le mode opératoire est le même que dans l'exemple 1. On obtient une émulsion lisse, douce et fraîche, non collante, ayant une viscosité d'environ 38 UD (soit 90 poises = 9 Pa.s) (mesurée à environ 25°C avec un viscosimètre Rhéomat Mettler, mobile 4).

### Exemples comparatifs :

On a remplacé dans l'exemple 2 l'Hostacerin AMPS par des agents gélifiants habituellement utilisés dans les compositions cosmétiques, en une quantité équivalente :
- Le carbopol (commercialisé par la société Goodrich) est un polymère carboxyvinylique (nom CTFA : carbomer).
- Le Sepigel 305 (commercialisé par la société SEPPIC) est une émulsion inverse de copolymère réticulé obtenu à partir d'acrylamide et de sel de sodium d'acide 2-acrylamido-2-methylpropanesulfonique, dans un rapport en % de mole d'environ 70/30 à 30/70 et de préférence de 60/40 à 40/60, et d'un monomère polyfonctionnel comme agent réticulant. Ce copolymère est plus particulièrement décrit dans le document EP-A-503853.
- Le Pemulen (commercialisé par la société Goodrich) est un copolymère d'acide ou d'anhydride carboxylique monooléfiniquement insaturé en C₃-C₆ et d'ester d'acide acrylique comportant une chaine grasse ayant de 10 à 30 atomes de carbone (nom CTFA : acrylates/C10-C30 alkylacrylate crosspolymer).

Le tableau indiqué ci-dessus montre nettement les avantages de la composition selon l'invention par rapport aux compositions contenant à la place du polymère utilisé selon l'invention, un gélifiant habituellement utilisé dans les compositions cosmétiques.

**Tableau**

| Composition | Exemple 2 selon l'invention | Exemple comparatif 1 | Exemple comparatif 2 | Exemple comparatif 3 |
|---|---|---|---|---|
| Cyclohexaméthicone | 10 % | 10 % | 10 % | 10 % |
| Hydrocarbure ramifié | 5 % | 5 % | 5 % | 5 % |
| Silicone modifiée | 3 % | 3 % | 3 % | 3 % |
| Hostacerin AMPS | 1 % | - | - | - |
| Sepigel 305 | - | 2,5 % (soit 1 % de matière active) | - | - |
| Carbopol | - | - | 1% | - |
| Pemulen | - | - | - | 1 % |
| Conservateur | 0,4 % | 0,4 % | 0,4 % | 0,4 % |
| Eau | Qsp 100 % | Qsp 100 % | Qsp 100 % | Qsp 100 % |
| Viscosité à environ 25°C (Viscosimètre Rhéomat Mettler) | 90 poises (9 Pa.s) | 57 poises (5,7 Pa.s) | 52 poises (5,2 Pa.s) | Inférieure à 5 poises (< 5 Pa.s) |
| Aspect macroscopique | Emulsion lisse, douce, fraîche et non collante, reste stable après 2 mois à toutes températures après cycles de congélation. | Emulsion fluide, d'aspect huileux avec un relargage d'huile. Dephasage après 15 jours. | Emulsion fluide, toucher collant et poisseux, avec un effet rêche. Dephasage après cycles de congélation. | Emulsion très fluide, d'aspect hétérogène, pas lisse, mais granuleuse, au toucher collant. Dephasage après cycles de congélation. |
| Aspect au microscope | Emulsion régulière, aux bords nets | Emulsion irrégulière et grossière avec de larges plages de relargage | Dispersion grossière, rapidement déstabilisée avec séparation des phases | Dispersion hétérogène |

### Exemple 3 : Crème hydratante

### A. Phase huileuse

- Cyclométhicone 10 %
- Hydrocarbure ramifié 5 %
- Silicone modifiée (exemple 3 du brevet US-5,412,004) 5 %
   (soit 1,65 % de matière active)

### B. Phase aqueuse

- Hostacerin AMPS 1 %
- Glycérine 7 %
- Conservateur 0,4 %
- Eau qsp 100 %

Le mode opératoire est le même que dans l'exemple 1. On obtient une émulsion lisse, douce et fraîche, non collante.

### Exemple 4 : Démaquillant pour peaux sèches et sensibles

### A. Phase huileuse

- Cyclopentaméthicone 10 %
- Huile minérale 5 %
- Palmitate d'isopropyle 10 %
- Silicone modifiée (exemple 3 du brevet US-5,412,004) 10 %
   (soit 3,3 % de matière active)

### B. Phase aqueuse

- Hostacerin AMPS 0,5 %
- Glycérine 3 %
- Conservateur 0,4 %
- Eau qsp 100 %

Le mode opératoire est le même que dans l'exemple 1. On obtient une émulsion souple, douce et fraîche, facile à étaler. Cette émulsion a de bonnes propriétés de démaquillage et laisse la peau nette, fraîche et mate.

### Exemple 5 : Fond de teint matifiant

### A. Phase huileuse

- Cyclohexaméthicone 10 %
- Hydrocarbure ramifié 5 %
- Palmitate d'isopropyle 10 %
- Silicone modifiée (exemple 3 du brevet US-5,412,004) 15 %
   (soit 4,95 % de matière active)

### B. Phase des charges et pigments

- Pigments rouges, bruns, jaunes 5 %
- Amidon réticulé (DRY-FLO de la société National Starch) 5 %

### C. Phase aqueuse

- Hostacerin AMPS 1 %
- Glycérine 7 %
- Conservateur 0,4 %
- Eau qsp 100 %

Mode opératoire : on prépare séparément les phases aqueuse et huileuse. On disperse les pigments et charges dans la phase huileuse et on introduit la phase aqueuse gélifiée dans la phase huileuse sous agitation.

On obtient un fond de teint doux et très agréable à utiliser, donnant un aspect lisse et mat à la peau.

## Revendications

1. Composition comprenant, dans un milieu physiologiquement acceptable, une phase aqueuse dispersée dans une phase huileuse, **caractérisée en ce que** la phase huileuse comprend des particules d'un organopolysiloxane solide élastomère réticulé comportant au moins un groupement oxyalkyléné et **en ce que** la phase aqueuse comprend au moins un polymère poly(acide 2-acrylamido 2-méthylpropane sulfonique) réticulé et neutralisé.

2. Composition selon la revendication 1, **caractérisée en ce que** l'organopolysiloxane élastomère comporte au moins un groupement oxyéthyléné.

3. Composition selon la revendication 1 ou 2, **caractérisée en ce que** l'organopolysiloxane élastomère ne comporte que des groupements oxyéthylénés comme groupements oxyalkylénés.

4. Composition selon l'une quelconque des revendications précédentes, **caractérisée en ce que** l'organopolysiloxane élastomère est obtenu par réaction d'addition et de réticulation en milieu non aqueux, en présence d'un catalyseur, d'au moins :
- un premier organopolysiloxane (i) ayant au moins deux groupements vinyliques en position α-ω de la chaîne siliconée par molécule ; et
- un second organopolysiloxane (ii) ayant au moins un atome d'hydrogène lié à un atome de silicium par molécule et au moins un groupement oxyalkyléné.

5. Composition selon la revendication précédente, **caractérisée en ce que** le premier organopolysiloxane (i) est choisi parmi les polydiméthylsiloxanes.

6. Composition selon la revendication 4 ou 5, **caractérisée en ce que** le premier organopolysiloxane (i) est un α-ω-diméthylvinyl polydiméthylsiloxane.

7. Composition selon l'une des revendications 4 à 6, **caractérisée en ce que** le second organopolysiloxane (ii) est choisi parmi les polydiméthylsiloxanes ayant un ou plusieurs atomes d'hydrogène et un ou plusieurs groupements oxyalkylénés liés à un atome de silicium via un radical alkylène ayant de 1 à 22 atomes de carbone.

8. Composition selon l'une quelconque des revendications 4 à 7, **caractérisée en ce que** l'organopolysiloxane élastomère est sous forme d'un gel obtenu selon les étapes suivantes :
- (a) mélange du premier et second organopolysiloxanes (i) et (ii) ;
- (b) ajout d'une phase huileuse au mélange de l'étape (a) ;
- (c) polymérisation du premier et second organopolysiloxanes (i) et (ii) en phase huileuse en présence d'un catalyseur de platine.

9. Composition selon la revendication précédente, **caractérisée en ce que** le gel a un caractère élastique dominant aux faibles valeurs de la contrainte de cisaillement, défini comme suit : 800 Pa < G * ₚₗₐₜₑₐᵤ < 2 500 Pa avec δ ₚₗₐₜₑₐᵤ voisin de 10°, G* ₚₗₐₜₑₐᵤ représentant la consistance et δ ₚₗₐₜₑₐᵤ représentant l'élasticité, cette mesure étant faite à 1 Hz.

10. Composition selon l'une quelconque des revendications précédentes, **caractérisée en ce que** les particules d'organopolysiloxane élastomère ont une taille allant de 3 à 200 µm et de préférence de 3 à 50 µm.

11. Composition selon l'une des revendications précédentes, **caractérisée en ce que** l'organopolysiloxane élastomère est présent en une quantité en matière active allant de 0,1 à 10 % en poids par rapport au poids total de la composition.

12. Composition selon l'une quelconque des revendications précédentes, **caractérisée en ce que** le poly(acide 2-acrylamido 2-méthylpropane sulfonique) réticulé et neutralisé comprend, distribués de façon aléatoire :
a) de 90 à 99,9% en poids de motifs de formule générale (I) suivante : 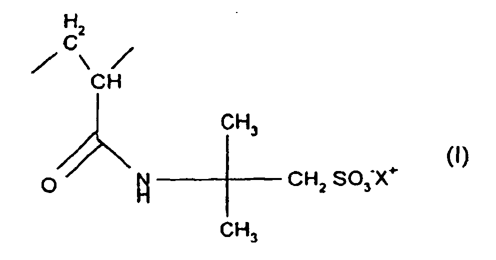 dans laquelle X⁺ désigne un cation ou un mélange de cations, au plus 10% mol des cations X⁺ pouvant être des protons H⁺ ;
b) de 0,01 à 10% en poids de motifs réticulants provenant d'au moins un monomère de réticulation ayant au moins deux doubles-liaisons oléfiniques ; les proportions en poids étant définis par rapport au poids total du polymère.

13. Composition selon la revendication précédente, **caractérisée en ce que** le poly(acide 2-acrylamido 2-méthylpropane sulfonique) comporte de 98 à 99,5 % en poids de motifs de formule (I) et de 0,2 à 2 % en poids de motifs réticulants.

14. Composition selon la revendication 12 ou 13, **caractérisée en ce que** dans la formule (1) le cation X⁺ est NH₄⁺ .

15. Composition selon l'une quelconque des revendications 12 à 14, **caractérisée en ce que** les monomères de réticulation répondent à la formule générale (II) suivante: 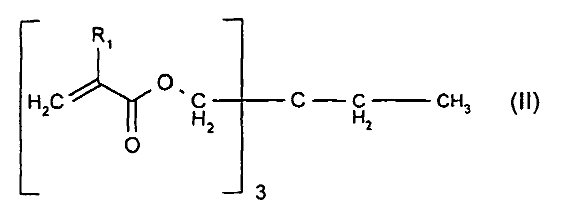 dans laquelle R₁ désigne hydrogène ou un alkyle en C₁-C₄.

16. Composition selon l'une quelconque des revendications 12 à 15, **caractérisée en ce que** le poly(acide 2-acrylamido 2-méthylpropane sulfonique) est réticulé par le triméthylol propane triacrylate.

17. Composition selon l'une quelconque des revendications précédentes,
**caractérisée en ce que** la quantité de poly(acide 2-acrylamido 2-méthylpropane sulfonique) va de 0,1 à 10 % en poids de matière active par rapport au poids total de la composition.

18. Composition selon l'une quelconque des revendications précédentes, **caractérisée en ce que** la phase huileuse est présente en une quantité allant de 5 à 50 % en poids par rapport au poids total de la composition.

19. Composition selon l'une quelconque des revendications précédentes, **caractérisée en ce que** la phase huileuse contient au moins une huile de silicone volatile.

20. Composition selon l'une quelconque des revendications précédentes, **caractérisée en ce que** la phase aqueuse représente au moins 65 % en poids par rapport au poids total de la composition.

21. Composition selon l'une quelconque des revendications précédentes,
**caractérisée en ce qu'**elle est exempte d'électrolyte.

22. Composition comprenant, dans un milieu physiologiquement acceptable, une phase aqueuse dispersée dans une phase huileuse, **caractérisée en ce que** la phase huileuse comprend des particules d'un organopolysiloxane solide élastomère réticulé comportant au moins un groupement oxyalkyléné et **en ce que** la phase aqueuse comprend au moins un polymère poly(acide 2-acrylamido 2-méthylpropane sulfonique) réticulé et neutralisé, la dite composition étant exempte d'actif instable en milieu oxydant en une teneur de 0,01 à 20 % du poids total de la composition.

23. Utilisation cosmétique de la composition selon l'une quelconque des revendications 1 à 22, pour le traitement, la protection, le soin, le démaquillage et/ou le nettoyage de la peau, des lèvres et/ou des cheveux, et/ou pour le maquillage de la peau et/ou des lèvres.

24. Procédé de traitement cosmétique de la peau, y compris du cuir chevelu, des cheveux, et/ou des lèvres, **caractérisé par le fait que** l'on applique sur la peau, les cheveux et/ou les lèvres, une composition selon l'une quelconque des revendications 1 à 22.

25. Utilisation de la composition selon l'une quelconque des revendications 1 à 22 pour la fabrication d'une crème destinée au traitement des peaux grasses.

26. Utilisation de l'association de particules d'un organopolysiloxane solide élastomère réticulé comportant au moins un groupement oxyalkyléné et d'au moins un polymère poly(acide 2-acrylamido 2-méthylpropane sulfonique) réticulé et neutralisé, pour obtenir une émulsion eau-dans-huile stable.

27. Utilisation de l'association de particules d'un organopolysiloxane solide élastomère réticulé comportant au moins un groupement oxyalkyléné et d'au moins un polymère poly(acide 2-acrylamido 2-méthylpropane sulfonique) réticulé et neutralisé, comme agent stabilisant d'une émulsion eau-dans-huile.

## Patentansprüche

1. Zusammensetzung, die in einem physiologisch akzeptablen Medium eine in einer Ölphase dispergierte wässerige Phase enthält, **dadurch gekennzeichnet, dass** die Ölphase Partikel eines vernetzten, elastomeren, festen Organopolysiloxans enthält, das mindestens eine alkoxylierte Gruppe aufweist, und **dadurch**, dass die wässerige Phase mindestens ein vernetztes und neutralisiertes Poly(2-acrylamido-2-methylpropansulfonsäure)-Polymer enthält.

2. Zusammensetzung nach Anspruch 1, **dadurch gekennzeichnet, dass** das elastomere Organopolysiloxan mindestens eine ethoxylierte Gruppe aufweist.

3. Zusammensetzung nach Anspruch 1 oder 2, **dadurch gekennzeichnet, dass** das elastomere Organopolysiloxan als alkoxylierte Gruppen nur ethoxylierte Gruppen enthält.

4. Zusammensetzung nach einem der vorhergehenden Ansprüche, **dadurch gekennzeichnet, dass** das elastomere Organopolysiloxan durch Addition und Vernetzung von zumindest den folgenden Verbindungen in einem nichtwässerigen Medium in Gegenwart eines Katalysators hergestellt wird:
- eines ersten Organopolysiloxans (i) mit mindestens zwei Vinylgruppen in α,ω-Stellung der Siliconkette pro Molekül; und
- eines zweiten Organopolysiloxans (ii) mit mindestens einem an ein Siliciumatom gebundenen Wasserstoffatom pro Molekül und mindestens einer alkoxylierten Gruppe.

5. Zusammensetzung nach dem vorhergehenden Anspruch, **dadurch gekennzeichnet, dass** das erste Organopolysiloxan (i) unter den Polydimethylsiloxanen ausgewählt ist.

6. Zusammensetzung nach Anspruch 4 oder 5, **dadurch gekennzeichnet, dass** das erste Organosiloxan (i) ein α,ω-Dimethylvinyl-polydimethylsiloxan ist.

7. Zusammensetzung nach einem der Ansprüche 4 bis 6, **dadurch gekennzeichnet, dass** das zweite Organopolysiloxan (ii) unter den Polydimethylsiloxanen ausgewählt ist, die ein' oder mehrere Wasserstoffatome und eine oder mehrere alkoxylierte Gruppen aufweisen, die über eine Alkylengruppe mit 1 bis 22 Kohlenstoffatomen an ein Siliciumatom gebunden sind.

8. Zusammensetzung nach einem der Ansprüche 4 bis 7, **dadurch gekennzeichnet, dass** das elastomere Organopolysiloxan in Form eines Gels vorliegt, das gemäß den folgenden Schritten hergestellt wird:
- (a) Mischen des ersten Organopolysiloxans (i) und des zweiten Organopolysiloxans (ii);
- (b) Zugabe einer Ölphase in das in Schritt (a) erhaltene Gemisch;
- (c) Polymerisation des ersten und zweiten Organopolysiloxans (i) und (ii) in der Ölphase in Gegenwart eines Platinkatalysators.

9. Zusammensetzung nach dem vorhergehenden Anspruch, **dadurch gekennzeichnet, dass** das Gel bei niedrigen Werten der Scherbeanspruchung einen dominierenden elastischen Charakter besitzt, der folgendermaßen definiert ist: 800 Pa < G*_{Plateau} < 2.500 Pa mit δ_{Plateau} in der Gegend von 10°, wobei G*_{Plateau} die Konsistenz und δ_{Plateau} die Elastizität angibt, wobei die Messung bei 1 Hz erfolgt.

10. Zusammensetzung nach einem der vorhergehenden Ansprüche, **dadurch gekennzeichnet, dass** die Partikel des elastomeren Organopolysiloxans eine Größe von 3 bis 200 µm und vorzugsweise 3 bis 50 µm besitzen.

11. Zusammensetzung nach einem der vorhergehenden Ansprüche, **dadurch gekennzeichnet, dass** das elastomere Organopolysiloxan in einer Wirkstoffmenge von 0,1 bis 10 Gew.-%, bezogen auf das Gesamtgewicht der Zusammensetzung, enthalten ist.

12. Zusammensetzung nach einem der vorhergehenden Ansprüche, **dadurch gekennzeichnet, dass** die vernetzte und neutralisierte Poly(2-acrylamido-2-methylpropan-sulfonsäure) zufällig verteilt enthält:
a) 90 bis 99,9 Gew.-% Einheiten der folgenden allgemeinen Formel (I): 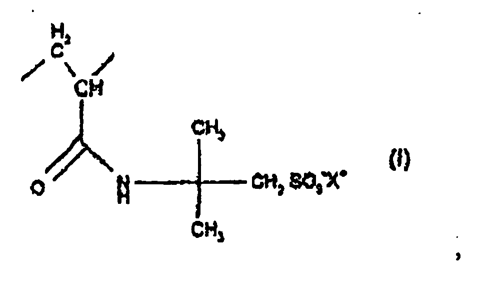 worin X⁺ ein Kation oder ein Gemisch von Kationen bedeutet, wobei höchstens 10 Mol-% der Kationen X⁺ Protonen H⁺ sein können;
b) 0,01 bis 10 Gew.-% Vernetzungseinheiten, die von mindestens einem Monomer zur Vernet2ung abgeleitet sind, das mindestens zwei olefinische Doppelbindungen aufweist; wobei die Gewichtsanteile bezogen auf das Gesamtgewicht des Polymers definiert sind.

13. Zusammensetzung nach dem vorhergehenden Anspruch, **dadurch gekennzeichnet, dass** die Poly-(2-acrylamido-2-methylpropan-sulfonsäure) 98 bis 99,5 Gew.-% Einheiten der Formel (I) und 0,2 bis 2 Gew.-% Vernetzungseinheiten aufweist.

14. Zusammensetzung nach Anspruch 12 oder 13, **dadurch** gekennzeichxlet, dass das Kation X⁺ in der Formel (I) NH₄⁺ bedeutet.

15. Zusammensetzung nach einem der Ansprüche 12 bis 14, **dadurch gekennzeichnet, dass** die vernetzenden Monomere der folgenden allgemeinen Formel (II) entsprechen: 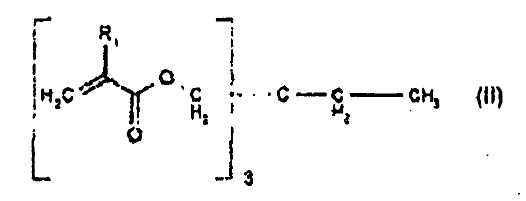 worin R₁ Wasserstoff oder C₁₋₄-Alkyl bedeutet.

16. Zusammensetzung nach einem der Ansprüche 12 bis 15, **dadurch gekennzeichnet, dass** die Poly-(2-acrylamido-2-methyl-propan-sulfonsäure) mit Trimethylolpropantriacrylat vernetzt ist.

17. Zusexnmensetzung nach einem der vorhergehenden Ansprüche, **dadurch gekennzeichnet, dass** der Mengenanteil der Poly-(2-acrylamido-2-methylpropan-sulfonsäure) im Bereich von 0,1 bis 10 Gew.-% wirksame Substanz, bezogen auf das Gesamtgewicht der Zusammensetzung, liegt.

18. Zusammensetzung nach einem der vorhergehenden Ansprüche, **dadurch gekennzeichnet, dass** die Ölphase in einer Menge von 5 bis 50 Gew.-%, bezogen auf das Gesamtgewicht der Zusammensetzung, enthalten ist.

19. Zusammensetzung nach einem der vorhergehenden Ansprüche, **dadurch gekennzeichnet, dass** die Ölphase mindestens ein flüchtiges Siliconöl enthält.

20. Zusammensetzung nach einem der vorhergehenden Ansprüche, **dadurch gekennzeichnet, dass** die wässerige Phase mindestens 65 Gew.-%, bezogen auf das Gesamtgewicht der Zusammensetzung, ausmacht.

21. Zusammensetzung nach einem der vorhergehenden Ansprüche, **dadurch gekennzeichnet, dass** sie keinen Elektrolyten enthält.

22. Zusammensetzung, die in einem physiologisch akzeptablen Medium eine in einer Ölphase dispergierte wässerige Phase aufweist, **dadurch gekennzeichnet, dass** die Ölphase Partikel eines vernetzten, elastomeren festen Organopolysiloxans enthält, das mindestens eine alkoxylierte Gruppe aufweist, und **dadurch**, dass die wässerige Phase mindestens ein vernetztes und neutralisiertes Poly(2-acrylamido-2-methylpropan-sulfonsäure)-polymer enthält, wobei die Zusammensetzung keinen in einem oxidierenden Medium instabilen Wirkstoff in einer Menge von 0,01 bis 20 Gew.-% des Gesamtgewichts der Zusammensetzung enthält.

23. Kosmetische Verwendung der Zusammensetzung nach einem der Ansprüche 1 bis 22 zur Behandlung, zum Schutz, zur Pflege, zum Abschminken und/oder zur Reinigung der Haut, der Lippen und/ oder der Haare und/ oder zum Schminken der Haut und/oder der Lippen.

24. Verfahren zur kosmetischen Behandlung der Haut einschließlich der Kopfhaut, der Haare und/oder der Lippen, **dadurch gekennzeichnet, dass** auf die Haut, die Haare und/oder die Lippen eine Zusammensetzung nach einem der Ansprüche 1 bis 22 aufgebracht wird.

25. Verwendung der Zusammensetzung nach einem der Ansprüche 1 bis 22 für die Herstellung einer Creme, die zur Behandlung von fettiger Haut vorgesehen ist.

26. Verwendung der Kombination aus Partikeln eines vernetzten, elastomeren, festen Organopolysiloxans, das mindestens eine alkoxylierte Gruppe aufweist, und mindestens eines vernetzten und neutralisierten Poly(2-acrylamido-2-methylpropan-sulfonsäure)-Polymers zur Herstellung einer stabilen Wasser-in-Öl-Emulsion.

27. Verwendung der Kombination aus Partikeln eines vernetzten, elastomeren, festen Organopolysiloxans, das mindestens eine alkoxylierte Gruppe enthält, und mindestens eines vernetzten und neutralisierten Poly(2-acrylamido-2-methylpropan-sulfonsäure)-Polymers als Stabilisierungsmittel für eine Wasserin-Öl-Emulsion.

## Claims

1. Composition comprising, in a physiologically acceptable medium, an aqueous phase dispersed in an oily phase, **characterized in that** the oily phase comprises particles of a crosslinked solid organopolysiloxane elastomer comprising at least one oxyalkylene group and **in that** the aqueous phase comprises at least one crosslinked and neutralized poly(2-acrylamido-2-methylpropanesulphonic acid) polymer.

2. Composition according to Claim 1, **characterized in that** the organopolysiloxane elastomer comprises at least one oxyethylene group.

3. Composition according to Claim 1 or 2, **characterized in that** the organopolysiloxane elastomer only comprises oxyethylene groups as oxyalkylene groups.

4. Composition according to any one of the preceding claims, **characterized in that** the organopolysiloxane elastomer is obtained by an addition and crosslinking reaction in a non-aqueous medium, in the presence of a catalyst, of at least:
- one first organopolysiloxane (i) having at least two vinyl groups in the α,ω-position of the silicone chain per molecule; and
- one second organopolysiloxane (ii) having at least one hydrogen atom bonded to a silicon atom per molecule and at least one oxyalkylene group.

5. Composition according to the preceding claim, **characterized in that** the first organopolysiloxane (i) is chosen from polydimethylsiloxanes.

6. Composition according to Claim 4 or 5, **characterized in that** the first organopolysiloxane (i) is an α,ω-dimethylvinylpolydimethylsiloxane.

7. Composition according to one of Claims 4 to 6, **characterized in that** the second organopolysiloxane (ii) is chosen from polydimethylsiloxanes having one or more hydrogen atoms and one or more oxyalkylene groups bonded to a silicon atom via an alkylene radical having from 1 to 22 carbon atoms.

8. Composition according to any one of Claims 4 to 7, **characterized in that** the organopolysiloxane elastomer is in the form of a gel obtained according to the following stages:
- (a) mixing the first and second organopolysiloxanes (i) and (ii) ;
- (b) adding an oily phase to the mixture of stage (a);
- (c) polymerizing the first and second organopolysiloxanes (i) and (ii) in the oily phase in the presence of a platinum catalyst.

9. Composition according to the preceding claim, **characterized in that** the gel has a dominant elastic nature at low values of the shear stress defined as follows: 800 Pa < G*ₚₗₐₜₑ < 2 500 Pa with δₚₗₐₜₑ in the region of 10°, G*ₚₗₐₜₑ representing the consistency and δₚₗₐₜₑ representing the elasticity, this measurement being made at 1 Hz.

10. Composition according to any one of the preceding claims, **characterized in that** the organopolysiloxane elastomer particles have a size ranging from 3 to 200 µm and preferably from 3 to 50 µm.

11. Composition according to one of the preceding claims, **characterized in that** the organopolysiloxane elastomer is present in an amount, as active material, ranging from 0.1 to 10% by weight with respect to the total weight of the composition.

12. Composition according to any one of the preceding claims, **characterized in that** the crosslinked and neutralized poly(2-acrylamido-2-methylpropanesulphonic acid) comprises, distributed randomly:
a) from 90 to 99.9% by weight of units of following general formula (I): 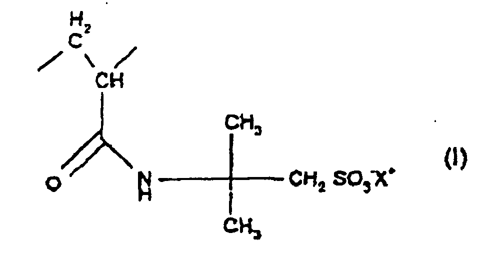 in which X⁺ denotes a cation or a mixture of cations, it being possible for at most 10 mol% of the X⁺ cations to be H⁺ protons;
b) from 0.01 to 10% by weight of crosslinking units originating from at least one crosslinking monomer having at least two olefinic double bonds; the proportions by weight being defined with respect to the total weight of the polymer.

13. Composition according to the preceding claim, **characterized in that** the poly(2-acrylamido-2-methylpropanesulphonic acid) comprises from 98 to 99.5% by weight of units of formula (I) and from 0.2 to 2% by weight of crosslinking units.

14. Composition according to Claim 12 or 13, **characterized in that**, in the formula (I), the X⁺ cation is NH₄⁺.

15. Composition according to any one of Claims 12 to-14, **characterized in that** the crosslinking monomers correspond to the following general formula (II): 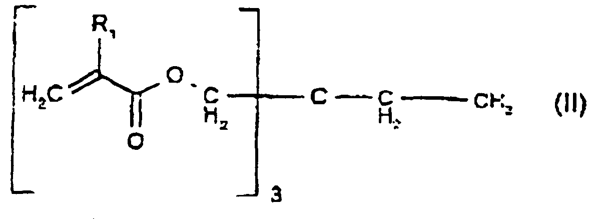 in which R₁ denotes hydrogen or a C₁-C₄ alkyl.

16. Composition according to any one of Claims 12 to 15, **characterized in that** the poly(2-acrylamido,-2-methylpropanesulphonic acid) is crosslinked with trimethylolpropane triacrylate.

17. Composition according to any one of the preceding claims, **characterized in that** the amount of poly(2-acrylamido-2-methylpropanesulphonic acid) ranges from 0.1 to 10% by weight of active material with respect to the total weight of the composition.

18. Composition according to any one of the preceding claims, **characterized in that** the oily phase is present in an amount ranging from 5 to 50% by weight with respect to the total weight of the composition.

19. Composition according to any one of the preceding claims, **characterized in that** the oily phase comprises at least one volatile silicone oil.

20. Composition according to any one of the preceding claims, **characterized in that** the aqueous phase represents at least 65% by weight with respect to the total weight of the composition.

21. Composition according to any one of the preceding claims, **characterized in that** it is devoid of electrolyte.

22. Composition comprising, in a physiologically acceptable medium, an aqueous phase dispersed in an oily phase, **characterized in that** the oily phase comprises particles of a crosslinked solid organopolysiloxane elastomer comprising at least one oxyalkylene group and **in that** the aqueous phase comprises at least one crosslinked and neutralized poly(2-acrylamido-2-methylpropanesulphonic acid) polymer, the said composition being devoid of active principle unstable in an oxidizing environment at a content of 0.01 to 20% of the total weight of the composition.

23. Cosmetic use of the composition according to any one of Claims 1 to 22 in treating, protecting, caring for, removing make-up from and/or cleansing the skin, lips and/or hair and/or in making up the skin and/or lips.

24. Process for the cosmetic treatment of the skin, including the scalp, the hair and/or the lips, **characterized in that** a composition according to any one of Claims 1 to 22 is applied to the skin, the hair and/or the lips.

25. Use of the composition according to any one of Claims 1 to 22 in the manufacture of a cream intended for the treatment of greasy skin.

26. Use of the combination of particles of a crosslinked solid organopolysiloxane elastomer comprising at least one oxyalkylene group and of at least one crosslinked and neutralized poly(2-acrylamido-2-methylpropanesulphonic acid) polymer for obtaining a stable water-in-oil emulsion.

27. Use of the combination of particles of a crosslinked solid organopolysiloxane elastomer comprising at least one oxyalkylene group and of at least one crosslinked and neutralized poly(2-acrylamido-2-methylpropanesulphonic acid) polymer as agent for stabilizing a water-in-oil emulsion.
